# EUROPÄISCHE PATENTSCHRIFT

(11) **EP 2 339 993 B1**
(45) Veröffentlichungstag und Bekanntmachung des Hinweises auf die Patenterteilung: **27.08.2014**
(21) Anmeldenummer: 09740261.4
(22) Anmeldetag: 24.09.2009
(51) Int. Cl.: A61F 2/44

(54) **BAUSATZ ZUM AUFBAU EINER BANDSCHEIBENPROTHESE SOWIE SYSTEM ZUM AUFBAU VERSCHIEDENER BANDSCHEIBENPROTHESEN**
KIT FOR CONSTRUCTING A SPINAL DISK PROSTHESIS, AND SYSTEM FOR CONSTRUCTING DIFFERENT SPINAL DISK PROSTHESES
JEU DE PIÈCES POUR LA CONSTRUCTION D'UNE PROTHÈSE DE DISQUE INTERVERTÉBRAL ET SYSTÈME DE CONSTRUCTION DE DIFFÉRENTES PROTHÈSES DE DISQUES INTERVERTÉBRAUX

(30) Priorität: 24.09.2008 DE 102008048739
(43) Veröffentlichungstag der Anmeldung: 06.07.2011
(73) Patentinhaber: Copf, Franz, 70184 Stuttgart (DE)
(72) Erfinder: Copf, Franz, 70184 Stuttgart (DE)
(74) Vertreter: Schwanhäußer, Gernot
(86) Internationale Anmeldenummer: PCT/EP2009/006906
(87) Internationale Veröffentlichungsnummer: WO 2010/034496

(56) Entgegenhaltungen:
- WO-A-2007/003438
- WO-A-2009/071044
- US-A1- 2003 074 070
- US-A1- 2007 055 378

## Beschreibung

### 1. Gebiet der Erfindung

Die Erfindung betrifft einen Bausatz zum Aufbau einer Bandscheibenprothese, die zur Anordnung in einem von Wirbelkörpern begrenzten Bandscheibenfach vorgesehen ist, nach dem Oberbegriff des Anspruchs 1. Ein solcher Bausatz ist aus der US 2007/0055378 A1 bekannt.

Die Erfindung betrifft ferner ein System zum Aufbau unterschiedlich konfigurierter derartiger Bandscheibenprothesen nach dem Oberbegriff des Anspruchs 9. Ein solches System ist aus der WO 2007/003438 A2 bekannt.

### 2. Beschreibung des Standes der Technik

Bandscheibenprothesen werden bei Bandscheibenschäden implantiert, bei denen ein Ersatz der beschädigten Bandscheibe notwendig ist. Dabei wird die Bandscheibenprothese in ein Bandscheibenfach der Wirbelsäule eingesetzt, das durch zwei benachbart angeordnete Wirbelkörper begrenzt ist.

Bandscheibenprothesen sollen eine möglichst geringe Beeinträchtigung der Beweglichkeit des Patienten gewährleisten und sind häufig als gelenkig miteinander verbundene Prothesenplatten ausgeführt. Um eine vorteilhafte Anpassung der Bandscheibenprothese an die anatomischen Gegebenheiten zu ermöglichen, ist es aus der vorstehend erwähnten WO 2007/003438 A2 bekannt, Bandscheibenprothesen in unterschiedlichen Konfigurationen bereitzustellen. Die Konfigurationen unterscheiden sich z.B. hinsichtlich der Größe, der Prothesenplatten, des Abstands zwischen den Prothesenplatten und der Lage des Bewegungszentrums, d.h. Drehpunkte, um den die Prothesenplatten zueinander verschwenkt werden können. Hierzu weist die Bandscheibenprothese Zwischenelemente auf, die als. Gelenkelemente oder Schwenkwinkelbegrenzer ausgeführt sein können. Zu diesem Zweck sind in den Prothesenplatten Aufnahmeschächte vorgesehen, die zur Aufnahme der Zwischenelemente ausgebildet sind. Mit den austauschbaren Zwischenelementen kann der Abstand und der maximale Schwenkwinkel zwischen den Prothesenplatten eingestellt und die Lage des Bewegungszentrums festgelegt werden.

Ein Drehlager der bekannten Bandscheibenprothese ist als Kugelgelenk ausgebildet und weist im implantierten Zustand im Bandscheibenfach drei rotatorische Freiheitsgrade der Bewegung auf. Die Bewegungseigenschaften der ursprünglich aus den gegenüberliegenden Wirbelkörpern und der Bandscheibe gebildeten kleinsten funktionellen Einheit der Wirbelsäule, die auch als Junghanssches Bewegungssegment oder Functional Spinal Unit (FSU) bezeichnet wird, soll durch die Bandscheibenprothese zumindest weitestgehend nachgebildet werden. Die Bandscheibenprothese soll wie die ursprüngliche Bandscheibe eine Beugung nach vorne, die auch als Flexion bezeichnet wird, eine Streckung nach hinten, die auch als Extension bezeichnet wird, eine Seitenneigung nach rechts oder links, die auch als Lateralflexion und eine Drehbewegung, die im Wesentlichen um eine sich längs der Wirbelsäule erstreckende Drehachse stattfindet und die auch als Rotation bezeichnet wird, ermöglichen.

Die natürliche Bandscheibe, die aus einem äußeren, elastischen Bindegewebering und einem davon umschlossenen weichen Kern besteht, dient als elastisches Gelenk- und Dämpfungselement und wird bei den Relativbewegungen der Wirbelkörper deformiert. Bedingt durch ihren Aufbau hat die Bandscheibe neben der Funktion als Gelenkelement auch eine Funktion als Ausschlagbegrenzung.für die Relativbewegungen der Wirbelkörper. Aus der bereits erwähnten WO 2007/003438 A2 ist es bekannt, an gegenüberliegenden Prothesenplatten austauschbare Anschläge anzuordnen, die eine Begrenzung der Schwenkbewegung der Prothesenplatten bei einer Seitenneigung (Lateralflexion) nach rechts oder links ermöglichen.

Aus der US 2008/0221693 A1 ist eine Bandscheibenprothese bekannt, bei der die ein Kugelgelenk bildenden Gelenkelemente einstückig an den Prothesenplatten ausgebildet sind. Überstände, die an einer Prothesenplatte im Bereich einer Kugelkalotte ausgebildet sind, greifen dabei in Ausnehmungen ein, die bei der anderen Prothesenplatte im Bereich einer zur Kugelkalotte komplementären Hohlkugelschale ausgebildet sind. Die Überständen bilden mit den Ausnehmungen eine Begrenzungseinrichtung, welche dazu dient, eine Drehbewegung um eine im Wesentlichen normal zu den Außenflächen der Prothesenplatten ausgerichteten Drehachse zu begrenzen. Allerdings lässt sich der zugelassene maximale Drehwinkel bei dieser bekannten Bandscheibenprothese nicht verändern.

Aus der US 2005/0234553 A1 ist eine Bandscheibenprothese bekannt, die einen elastischen, insgesamt topfförmigen Körper aufweist, dessen Wandung mit einem schraubenförmig umlaufenden Schlitz versehen ist. Am Boden des topfförmigen Körpers ist ein erstes Gelenkelement befestigt, das mit einem zweiten Gelenkelement ein Kugelgelenk bildet. Das zweite Gelenkelement ist dabei seinerseits umfangsseitig oben am Innenrand des topfförmigen Körpers befestigt. Einer relativen Verdrehung der Gelenkelemente um eine senkrecht zum Topfboden verlaufende Achse setzt der mit den Gelenkelementen fest verbundene Körper einen Torsionswiderstand entgegen, der zu einer Begrenzung der Drehbewegung führt. Der sehr einfache Aufbau dieser bekannten Bandscheibenprothese bringt es jedoch mit sich, dass man auch diese Begrenzungseinrichtung für die Drehbewegung nicht unabhängig von der Dimensionierung der Bandscheibenprothese einstellen kann.

Aus der eingangs bereits genannten US 2007/0055378 A1 ist eine Bandscheibenprothese bekannt, bei der eine Drehwinkelbegrenzung durch zwei an den Prothesenplatten festgelegten Zwischenelementen erreicht wird. Ein Zwischenelement ist dabei mit Nasen versehen, die jeweils in eine Aussparung eingreifen, die an dem anderen Zwischenelement ausgebildet ist.

### ZUSAMMENFASSUNG DER ERFINDUNG

Aufgabe der Erfindung ist es, einen Bausatz zum Aufbau einer Bandscheibenprothese anzugeben, mit dem sich der maximal zulässige Drehwinkel einstellen lässt, und zwar unabhängig von der Konfiguration der Prothesenplatten, deren Dimensionierung idealerweise von vielen unterschiedlichen Faktoren abhängig gemacht werden sollte.

Gelöst wird diese Aufgabe durch einen Bausatz mit den Merkmalen des Anspruchs 1.

Der Erfinder hat erkannt, dass nur dann, wenn die Begrenzungseinrichtung zur Begrenzung der Drehbewegung als eigenständiges, mit den Prothesenplatten verbindbares Bauteil ausgebildet ist, der maximal zulässige Drehwinkel unabhängig von der Ausbildung der Prothesenplatten festgelegt werden kann. Auf diese Weise reduziert sich die Zahl der vorzuhaltenden Bauteile erheblich, wenn man Bandscheibenprothesen bereitstellen möchte, die unterschiedliche maximale Drehwinkel zulassen, aber auch unterschiedlich konfigurierte Prothesenplatten aufweisen können. Soll beispielsweise eine Auswahl aus vier unterschiedlichen maximalen Drehwinkeln und vier unterschiedlichen Geometrien für Prothesenplatten möglich sein, so genügt es erfindungsgemäß, vier unterschiedliche Begrenzungseinrichtungen sowie vier unterschiedliche Sätze von Prothesenplatten bereitzustellen. Bei solchen bekannten Bandscheibenprothesen hingegen, bei denen die Drehwinkelbegrenzung stets integraler Teil der Prothesenplatten ist, müssen insgesamt 16 unterschiedliche Sätze von derartigen Teilen bereitgestellt werden, um die gleichen Auswahlmöglichkeiten zu erhalten. Dies erhöht die Kosten für die Vorhaltung und Desinfektion der Prothesen erheblich.

Die Außenflächen der Prothesenplatten liegen nach der Implantation der Bandscheibenprothese in das Bandscheibenfach im Wesentlichen flächig an den einander zugewandten Oberflächen benachbarter Wirbelkörper an. Die Oberflächen der Wirbelkörper sind in einer Neutrallage der Wirbelsäule im Wesentlichen parallel zueinander ausgerichtet. Außerdem sind die Oberflächen der Wirbelkörper in der Neutrallage der Wirbelsäule normal zu einem Abschnitt einer bei seitlicher Betrachtung der Wirbelsäule S-förmig geschwungenen, bei rückwärtiger Betrachtung der Wirbelsäule im Wesentlichen geraden Mittelachse der Wirbelsäule ausgerichtet. Benachbarte Wirbelkörper werden bei einer Drehbewegung der Wirbelsäule aus der Neutrallage im Wesentlichen um die Mittelachse der Wirbelsäule relativ zueinander verdreht. Somit ergibt sich, dass die Prothesenplatten bei einer derartigen Drehbewegung relativ zueinander in einer Drehebene bewegt werden, die im Wesentlichen normal zur Mittelachse der Wirbelsäule ausgerichtet ist. Die Mittelachse der Wirbelsäule entspricht somit im Wesentlichen der Drehachse der Prothesenplatten.

Mit dem erfindungsgemäßen Bausatz lässt sich ein System zum Aufbau verschiedener Bandscheibenprothesen realisierten, das aufweist:
a) einen Satz von unterschiedlich konfigurierten Paaren relativbeweglich zueinander anordenbarer Prothesenplatten, deren Außenflächen zur Anlage an Oberflächen gegenüberliegender Wirbelkörper vorgesehen sind und deren Innenflächen nach dem Zusammensetzen korrespondierende Lagerflächen eines Drehlagers bilden, und
b) einen Satz von dem Drehlager zugeordneten Begrenzungseinrichtungen zur Begrenzung einer Drehbewegung der Prothesenplatten um eine im Wesentlichen normal zu den Außenflächen der Prothesenplatten ausgerichtete Drehachse, wobei sich die Begrenzungseinrichtungen des Satzes zumindest dadurch voneinander unterscheiden, dass sie die Drehbewegung auf unterschiedliche maximale Drehwinkel begrenzen, und wobei die Begrenzungseinrichtungen mit unterschiedlichen Prothesenplatten verbindbar sind.

Im Prinzip ist es möglich, die Begrenzungseinrichtung unlösbar mit den Prothesenplatten zu verbinden. In Betracht kommt beispielsweise ein nicht mehr ohne weiteres lösbarer Rastmechanismus oder auch eine kräftschlüssige Verbindung, etwa durch Kleben. Bei einer bevorzugten Ausgestaltung der Erfindung ist die Begrenzungseinrichtung jedoch lösbar mit den Prothesenplätten verbindbar.. Dies hat zum einen den Vorteil, dass eine versehentlich mit den Prothesenplatten verbundene Begrenzungseinrichtung nicht dazu führt, dass die dadurch entstandene Baugruppe zumindest für den betreffenden Patienten nicht mehr eingesetzt werden kann. Zum anderen ermöglicht es eine lösbare Verbindung bei geeigneter Ausbildung der Begrenzungseinrichtung, diese gegebenenfalls auch nach der Implantierung der Bandscheibenprothese im Bandscheibenfach noch gegen eine andere Begrenzungseinrichtung auszutauschen. Ein solcher Bedarf kann beispielsweise entstehen, wenn ein zunächst zu großzügig bemessener maximaler Drehwinkel zu einer Überlastung der angrenzenden Segmente der Wirbelsäule geführt hat. In diesem Fall ist es möglich, während einer Operation die Verbindung zu lösen, die Begrenzungseinrichtung zu entfernen und durch eine Begrenzungseinrichtung zu ersetzen, die nur noch einen kleineren maximalen Drehwinkel ermöglicht.

Bei einem anderen Ausführungsbeispiel ist die Begrenzungseinrichtung oder ein Teil davon mit einem Einschubelement verbunden, das in einen Aufnahmeschacht einführbar ist, der in einer der Prothesenplatten ausgebildet ist. Der Aufnahmeschacht kann dabei derart an das Einschubelement angepasst sein, dass das Einschubelement nur entlang einer Richtung in den Aufnahmeschacht einführbar ist.

Auf diese Weise kann der Chirurg die Bandscheibenprothese sehr einfach sogar während der Operation aus den Prothesenplatten und der Begrenzungseinrichtung zusammensetzen. Dies wiederum ermöglicht es, die Wahl der Begrenzungseinrichtung von Faktoren möglich machen, die der Chirurg erst während der Operation messen oder in sonstiger Weise feststellen kann.

Erfindungsgemäß umfasst die Begrenzungseinrichtung Bremsflächen, die derart ausgerichtet sind, dass bei einer Rotation einer Prothesenplatte um die Drehachse eine Bremsfläche, die an einer der zwei Prothesenplatten festgelegt ist, auf einer korrespondierenden Bremsfläche gleitet, die an der anderen Prothesenplatte festgelegt ist, wobei der Reibungswiderstand zwischen den Bremsflächen infolge des von den Wirbelkörpern ausgeübten Drucks mit zunehmendem Drehwinkel zunimmt.

Vorzugsweise sind die Bremsflächen in einer Neutralstellung der Bandscheibenprothese derart voneinander beabstandet, dass eine Seitenneigung (Lateralflexion) in einem vorgegebenen Schwenkwinkelbereich möglich ist.

Bei einer Weiterbildung sind die Bremsflächen derart ausgebildet, dass sich mit zunehmendem Drehwinkel der Abstand zwischen den Prothesenplatten vergrößert. Bei einer Abstandsvergrößerung der Prothesenplatten wird der um die Wirbelsäule herum angeordnete Bandapparat gedehnt und führt zu einer zusätzlichen Erhöhung des von den Wirbelkörpern ausgeübten Drucks. Dadurch wird auch der Reibungswiderstand zwischen den Bremsflächen erhöht. Unter Ausnutzung der anatomischen Gegebenheiten des Patienten wird auf diese Weise eine der Drehung entgegenwirkende Abbremsung erzielt, die kräftig, jedoch stets kontinuierlich mit zunehmendem Drehwinkel ansteigt und dadurch kurzzeitige Belastungsspitzen, wie sie etwa bei ungedämpften Anschlägen entstehen, vermeidet.

Die Bremsflächen können dabei derart ausgerichtet sein, dass die Bremsfläche auf einer Prothesenplatte auf der korrespondierenden Bremsfläche auf.der anderen Prothesenplatte erst nach Überschreiten eines vorgegebenen Drehwinkels aufgleitet. In diesem Falle tritt die Bremswirkung somit erst ab einem gewissen Drehwinkel ein. Dieses Verhalten entspricht demjenigen der natürlichen Bandscheibenprothese, da auch dort bei kleinem Drehwinkel die Bandscheibe nur einen äußerst geringen Widerstand gegen die Drehung aufbringt.

Günstig ist es, wenn die Bremsflächen nicht Teil des Drehlagers sind.. Auf diese Weise ist sichergestellt, dass ein etwaiger Abrieb oder sonstiger Verschleiß der Bremsflächen sich nicht ungünstig auf die Eigenschaften des Drehlagers auswirken kann.

Bei einem anderen Ausführungsbeispiel umfasst die Begrenzungseinrichtung wenigstens zwei gegenüberliegenden Prothesenplatten zugeordnete Anschlagflächen zur Drehwinkelbegrenzung. Damit kann eine besonders einfache Aufbauweise für die Bandscheibenprothese verwirklicht werden.

Bei einer Weiterbildung der Erfindung ist vorgesehen, dass die Begrenzungseinrichtung für die Bereitstellung einer drehwinkelabhängigen Bremskraft in einem Drehwinkelbereich eingerichtet ist, der wenigstens 10 Prozent, vorzugsweise wenigstens 20 Prozent, eines vorgebbaren Maximaldrehwinkels der Prothesenplatten beträgt. Der Maximaldrehwinkel ist konstruktiv durch die Gestaltung der Bandscheibenprothese vorgegeben. Der Maximaldrehwinkel ist der Winkel, der durch Verdrehung der implantierten Prothesenplatten aus einer Neutralstellung in eine der beiden Drehrichtungen überstrichen wird, bis es durch die Bremskraft oder das Bremsmoment zu einer vollständigen Abbremsung der Drehbewegung zwischen" den Prothesenplatten kommt. Die erfindungsgemäße Begrenzungseinrichtung stellt die Bremskraft oder das Bremsmoment zusätzlich zu einer ohnehin durch Reibung zwischen den Gelenkflächen der Prothesenplatten hervorgerufenen Reibkraft bereit. Die Bereitstellung der Bremskraft erfolgt erfindungsgemäß, wenn der Drehwinkel zwischen den Prothesenplatten einen Wert erreicht hat, der wenigstens 90 Prozent, bevorzugt wenigstens 80 Prozent, des Maximaldrehwinkels entspricht.

Vorteilhaft ist es, wenn die Begrenzungseinrichtung derart eingerichtet ist, dass der Maximaldrehwinkel und/oder die bereitgestellte Bremskraft für die Drehbewegung mit einem zwischen den Prothesenplatten eingeschlossenen Schwenkwinkel über eine, vorzugsweise stetig verlaufende, Kennlinie in Beziehung stehen. Eine Verschwenkung der Prothesenplatten erfolgt um Schwenkachsen des Drehlagers, die zumindest nahezu in der Drehebene der Drehbewegung liegen. Schwenkbewegungen der Prothesenplatten treten bei der Flexion, bei der Extension und bei der Lateralflexion der Wirbelsäule auf. Mit einer Schwenkbewegung der Prothesenplatten um wenigstens eine Schwenkachse findet bereits eine Dehnung der die Wirbelsäule umgebenden Bänder, Muskeln und Nerven statt. Wird der Schwenkbewegung noch eine Drehbewegung überlagert, so findet eine weitere Dehnung der Bänder, Muskeln und Nerven statt. Da die Dehnung einen vorgegebenen Wert nicht überschreiten soll, wird durch die Begrenzungseinrichtung sichergestellt, dass der Maximaldrehwinkel mit zunehmendem Schwenkwinkel / zunehmenden Schwenkwinkeln vierkleinert wird. Diese Charakteristik der Bandscheibenprothese bildet das Verhalten der natürlichen Bandscheibe ab, bei der die Torsionsspannungen durch die Verdrehung der .Wirbelkörper und der dazwischen angeordneten Bandscheibe den durch die Schwenkbewegung hervorgerufenen Druckspannungen in der Bandscheibe überlagert werden und somit zu einem erhöhten Bewegungswiderstand führen.

### KURZE BESCHREIBUNG DER ZEICHNUNGEN

Ausführungsbeispiele der Erfindung sind in den Zeichnungen dargestellt. Dabei zeigt:
- Figur 1: eine Draufsicht auf eine Prothesenplatte mit austauschbaren Anschlagelementen und einer austauschbaren Gelenkmulde,
- Figur 2: eine Seitenansicht der Prothesenplatte gemäß Figur 1,
- Figur 3: eine Draufsicht auf eine Prothesenplatte mit austauschbaren Anschlagelementen und einer austauschbaren Gelenkkugel,
- Figur 4: eine Seitenansicht der Prothesenplatte gemäß Figur 3,
- Figur 5: eine aus den Prothesenplatten gemäß den Figuren 1 und 3 gebildete Bandscheibenprothese,
- Figur 6: eine Draufsicht auf eine Prothesenplatte mit austauschbaren Anschlagelementen und einer austauschbaren Gelenkkugel, die zwei Anschlagvorsprünge aufweist,
- Figur 7: eine Schnittdarstellung der Prothesenplatte gemäß Figur 6,
- Figur. 8: eine Draufsicht auf eine Prothesenplatte mit austauschbaren Anschlagelementen und einer austauschbaren Gelenkmulde, die mit Bewegungsräumen für Anschlagvorsprünge versehen ist,
- Figur 9: eine Schnittdarstellung der Prothesenplatte gemäß Figur 8,
- Figur 10: eine aus den Prothesenplatten gemäß den Figuren 6 und 8 gebildete Bandscheibenprothese.

In allen Figuren sind funktionsgleiche Bauteile mit den gleichen Bezugszeichen versehen.

### BESCHREIBUNG BEVORZUGTER AUSFÜHRUNGSBEISPIELE

Die nachfolgend beschriebenen Ausführungsbeispiele für Bandscheibenprothesen umfassen jeweils Prothesenplatten 10, die aus einem metallischen Werkstoff hergestellt sind und eine nierenförmige Außenkontur aufweisen. Eine Außenfläche 12 der Prothesenplatten 10 dient zur Anlage an einem nicht dargestellten Wirbelkörper und ist mit einer Wölbung 16 versehen, welche in einen aus festerem Knochenmaterial gebildeten Ring des Wirbelkörper eingreift. Eine Innenfläche 14 der Prothesenplatten 10 weist drei Schächte 18, 20, 22 auf, die zur Aufnahme von Einschubelementen eingerichtet sind. Andere Ausgestaltungen von Prothesenplatten können ebenfalls zur Verwirklichung der erfindungsgemäßen Merkmale eingesetzt werden.

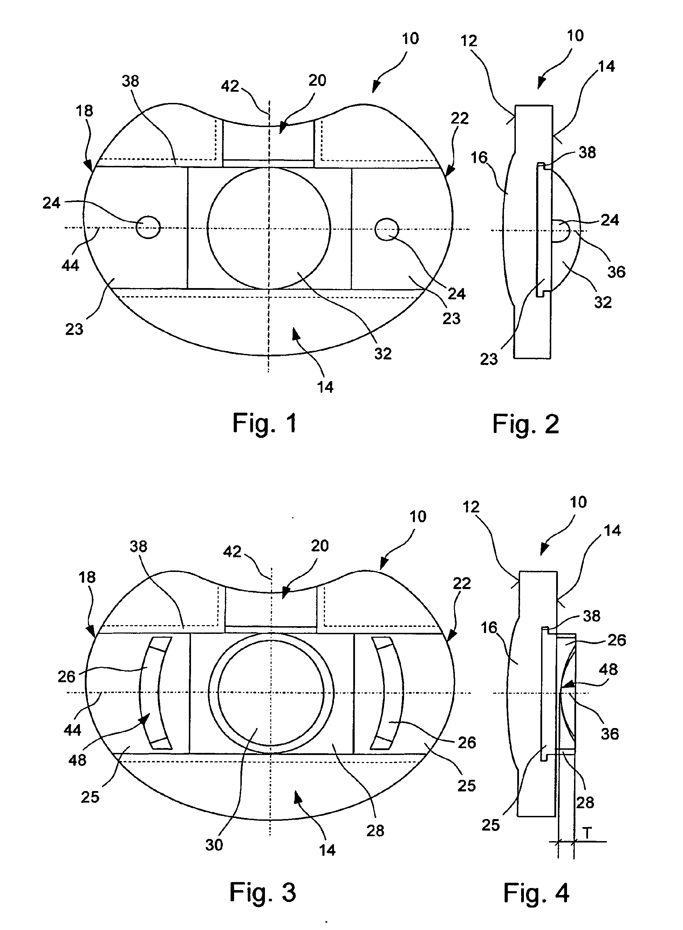

Bei der Prothesenplatte 10 gemäß den Figuren 1 und 2 sind in den Schächten 18 und 22 mit Bremszapfen 24 versehene Einschubelemente 23 aufgenommen. Bei der Prothesenplatte 10 gemäß den Figuren 3 und 4 sind in den Schächten 18 und 22 mit Bremsrampen 26 versehene Einschubelemente 25 aufgenommen.

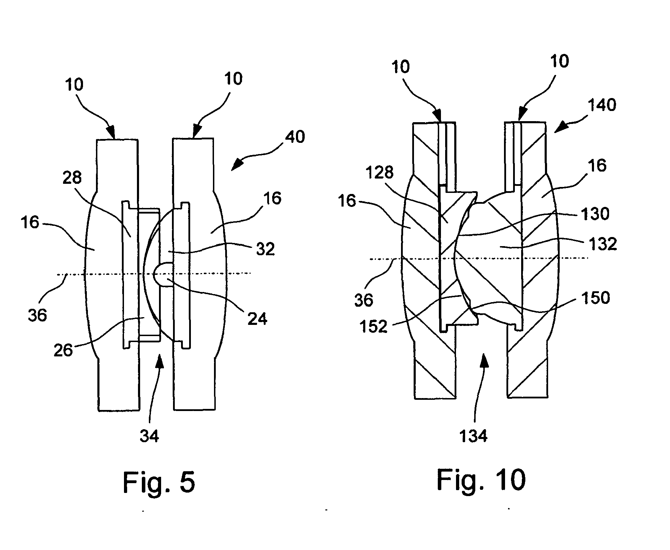

Bei der Prothesenplatte 10 gemäß den Figuren 3 und 4 ist im Schacht 20 eine Gelenkplatte 28 aufgenommen, die mit einer konkav geformten, kugelkalottenförmigen Gelenkmulde 30 versehen ist. Die Gelenkmulde 30 bildet mit der in der Prothesenplatte 10 gemäß den Figuren 1 und 2 angeordneten Gelenkkugel 32 ein in der Figur 5 näher dargestelltes Kugelgelenk 34. Das Kugelgelenk 34 ermöglicht eine Drehbewegung der Prothesenplatten 10 um die in der dargestellten Neutralstellung normal zu den Außenflächen 12 der Prothesenplatten 10 ausgerichtete Drehachse 36 sowie Schwenkbewegungen der Prothesenplatten 10 um Schwenkachsen 42 und 44, wie sie exemplarisch in den Figuren 1 und 3 dargestellt sind. Die Schwenkachsen 42 und 44 liegen in der in Figur 5 dargestellten Neutralstellung der Bandscheibenprothese 40 in einer orthogonal zur Drehachse 36 ausgerichteten Ebene. Somit ermöglicht das Kugelgelenk 34 eine Relativbewegung der Prothesenplatten 10 der Bandscheibenprothese 40 mit drei rotatorischen Freiheitsgraden.

Um eine zuverlässige Aufnahme der Einschubelemente 23, 25 in den Schächten 18, 20, 22 zu gewährleisten, sind dort jeweils in Randbereichen Nuten 38 vorgesehen, die einen rechteckigen Querschnitt aufweisen und die eine Festlegung der Einschubelemente 23, 25 in der Art einer Nut-Feder-Verbindung ermöglichen. Der Querschnitt der Nut 38 ist beispielsweise in der Figur 2 zu erkennen. Zusätzliche Maßnahmen zur Festlegung der Einschubelemente 23, 25 können in einer geeigneten Materialauswahl zur Erzielung einer hohen Reibung zwischen Einschubelement und Prothesenplatte 10 liegen. Ergänzend oder alternativ können auch nicht dargestellte Befestigungsmittel wie Schrauben eingesetzt werden, um die Einschubelemente 23, 25 zuverlässig an den Prothesenplatten 10 festzulegen.

Eine Schwenkwinkelbegrenzung für Schwenkbewegungen der Prothesenplatten 10 wird bereits durch deren äußere Gestalt gewährleistet, deren Innenflächen 14 sich bei Durchführung einer Schwenkbewegung bereichsweise annähern und zur Anlage kommen, so dass eine weitere Verschwenkung der Prothesenplatten 10 relativ zueinander unterbunden wird.

Eine Begrenzung der Drehbewegung der Prothesenplatten 10 um die Drehachse 36 allein aufgrund der Gestalt der Prothesenplatten 10 findet hingegen nicht statt. Zur Begrenzung der Drehbewegung dienen deswegen die mit Bremszapfen 24 und Bremsrampen 26 ausgerüsteten Einschubelemente 23, 25. Der. Bremszapfen 24 weist stirnseitig eine kugelkalottenförmige Gestalt auf. Die Bremsrampe 26 ist als gekrümmter Steg mit einer konkaven, wannenartigen Vertiefung 48 ausgebildet. Die Tiefe T der Vertiefung 48 und die Größe des Bremszapfens 24 sind derart gewählt, dass in der Neutralstellung gemäß Figur 5 sowohl eine Schwenkbewegung der Prothesenplatten 10 um die Schwenkachse 42 (Lateralflexion) als auch eine Drehung um.die.Drehachse 36 erfolgen kann. Gegenüber einer Schwenkbewegung um die Schwenkachse 44, die einer Flexion bzw. Extension der Wirbelsäule entspricht, ist die Anordnung aus Bremszapfen 24 und Bremsrampe 26 invariant.

Wie der Figur 5 entnommen werden kann, kommen bei einer relativen Verdrehung der Prothesenplatten 10 um die Drehachse 36 die Bremszapfen 24 in Anlage mit den Bremsrampen 26 und gleiten bei fortgesetzter Drehbewegung auf die mit zunehmender Steigung ansteigenden Bereiche der Bremsrampe 26 auf. Durch die achssymmetrische Ausbildung und Anordnung der Bremszapfen 24 und der Bremsrampen 26 bezogen auf die Schwenkachse 42 wird eine unerwünschte Verkippung der Prothesenplatten 10 vermieden. Bedingt durch die Druckkräfte, die von den Wirbelkörpern auf die Außenflächen der Prothesenplatten 10 eingeleitet werden, treten durch den Gleitvörgang der Bremszapfen 24 auf den Bremsrampen 26 mit zunehmendem Drehwinkel stetig ansteigende Reibungskräfte auf, die zu einer Abbremsung der Drehbewegung führen. Die Reibungskräfte rufen bezogen auf die Drehachse 36 ein Bremsmoment hervor, das über die Prothesenplatten 10 auf die Wirbelkörper eingeleitet wird und das dem durch den Bandapparat oder durch äußere Kräfte eingeleiteten Drehmoment entgegengesetzt ist.

Durch die wannenförmige Gestaltung der Vertiefungen 48 kann bei weiterer Vergrößerung des Drehwinkels eine Vergrößerung der Beabstandung der Prothesenplatten 10 hervorgerufen werden, da die Bremszapfen 24 auf die ansteigenden Bereiche der Bremsrampen 26 gleiten. Dadurch wird der umliegend an der Wirbelsäule angeordnete Bandapparat zusätzlich gespannt, womit die Druckkräfte auf die Prothesenplatten 10 und die daraus resultierenden Reibungskräfte zunehmen. Somit steigt das durch die Reibungskräfte hervorgerufene Bremsmoment mit zunehmendem Drehwinkel an.

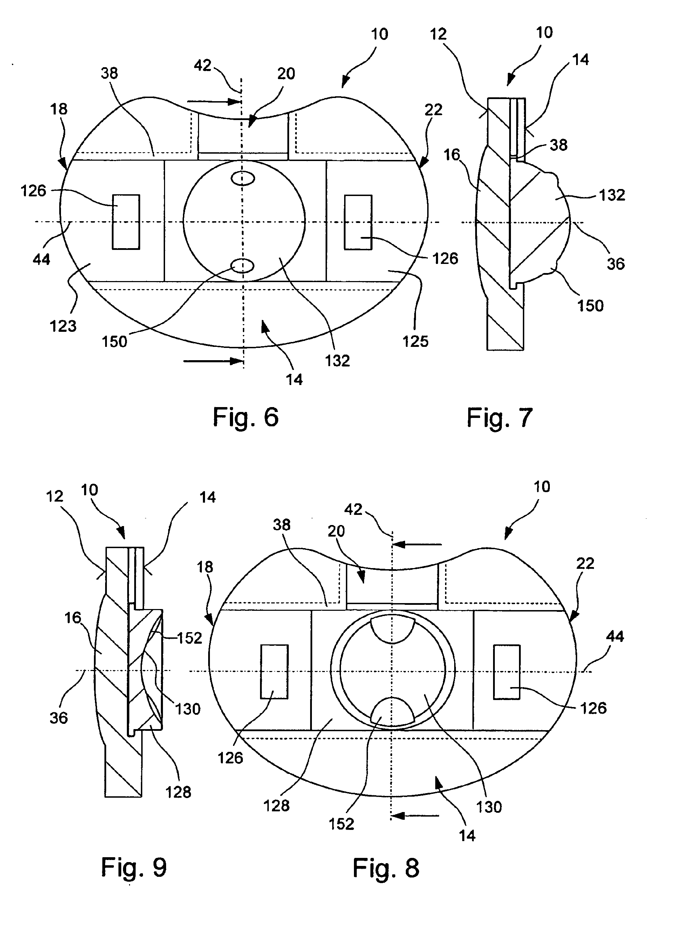

Bei dem in den Figuren 6 bis 10 dargestellten Ausführungsbeispiel einer Bandscheibenprothese 140 sind die Einschubelemente 123, 125 mit quaderförmigen Anschlägen 126 versehen, die zur Begrenzung einer Schwenkbewegung (Lateralflexion) um die Schwenkachse 42 dienen. Um eine Begrenzung des Drehwinkels zu erreichen, sind an der Gelenkkugel 132 zwei Vorsprünge 150 angebracht, die kugelkalottenförmig ausgebildet sind. An der Gelenkmulde 130 sind zu den Vorsprüngen 150 korrespondierende Steuermulden 152 vorgesehen, die der Drehwinkelbegrenzung dienen. Wie aus der Figur 10 hervorgeht, sind die Steuermulden 152 als gekrümmte Ausnehmungen in der Gelenkmulde 130 ausgebildet und ermöglichen somit sowohl eine Begrenzung einer Schwenkbewegung als auch eine Begrenzung einer Drehbewegung der Prothesenplatten 10. Dabei sind die Steuermulden 152 derart ausgebildet, dass eine relative Verschwenkung der Prothesenplatten 10 um die Schwenkachse 44 nicht beschränkt wird. Demgegenüber wird eine relative Verschwenkung der Prothesenplatten 10 um die Schwenkachse 42 auch ohne die in den Figuren 6 und 8 dargestellten quaderförmigen Anschläge 126 durch das Ansteigen der Steuermulden 152 bis hin zur Oberfläche der Gelenkmulde 130 begrenzt. Die Drehbewegung der Prothesenplatten 10 zueinander wird ebenfalls durch das Ansteigen der Steuermulden 152 bis hin zur Oberfläche der Gelenkmulde 130 begrenzt. Bei Annäherung der Vorsprünge 150 an die Randbereiche der Steuermulden 152 findet in ähnlicher Weise wie bei der Ausführungsform gemäß den Figuren 1 bis 5 eine Vergröβerung der Beabstandung der Prothesenplatten 10 statt, so dass dadurch die gewünschte Bremswirkung für die Drehbewegung hervorgerufen werden kann. Durch die Gestaltung der Kontur der Vorsprünge 150 und/oder der Steuermulden 152 kann Einfluss auf die Charakteristik der Drehwinkelbegrenzung und ggf. auf die Charakteristik der Schwenkwinkelbegrenzung genommen werden. Zur Beeinflussung dieser Charakteristika können die Größe der Vorsprünge 150 und/oder die Ausdehnung der Steuermulden 152 und deren Krümmungsverlauf variiert werden.

## Patentansprüche

1. Bausatz zum Aufbau einer Bandscheibenprothese, die zur Anordnung in einem von Wirbelkörpern begrenzten Bandscheibenfach vorgesehen ist, mit:
a) zwei relativbeweglich zueinander angeordneten Prothesenplatten (10), deren Außenflächen (12) zur Anlage an Oberflächen gegenüberliegender Wirbelkörper vorgesehen sind und deren Innenflächen (14) nach dem Zusammensetzen korrespondierende Lagerflächen eines Drehlagers (34; 134) bilden, und mit
b) einer dem Drehlager (34; 134) zugeordnete Begrenzungseinrichtung (24, 26; 150, 152) zur Begrenzung einer Drehbewegung um eine im Wesentlichen normal zu den Auβenflächen (12) der Prothesenplatten (10) ausgerichtete Drehachse (36), wobei die Begrenzungseinrichtung mit den Prothesenplatten (10) verbindbar ist,
**dadurch gekennzeichnet, dass**
die Begrenzungseinrichtung Bremsflächen (24, 26; 150, 152) umfasst, die derart ausgerichtet sind, dass bei einer Rotation einer Prothesenplatte (10) um die Drehachse (36) eine Bremsfläche (24, 26; 150, 152), die an einer der zwei Prothesenplatten (10) festgelegt ist, auf einer korrespondierenden Bremsfläche gleitet, die an der anderen Prothesenplatte festgelegt ist, wobei der Reibungswiderstand zwischen den Bremsflächen infolge des von den Wirbelkörpern ausgeübten Drucks mit zunehmendem Drehwinkel zunimmt.

2. Bausatz nach Anspruch 1, **dadurch gekennzeichnet, dass** die Begrenzungseinrichtung (24, 26; 150, 152) lösbar mit den Prothesenplatten (10) verbindbar ist.

3. Bausatz nach Anspruch 1 oder 2, **dadurch gekennzeichnet, dass** die Begrenzungseinrichtung (24, 26; 150, 152) oder ein Teil davon mit einem Einschubelement (23, 25; 128) verbunden ist, das in einen Aufnahmeschacht (18, 22) einführbar ist, der in einer der Prothesenplatten (10) ausgebildet ist.

4. Bausatz nach Anspruch 3, **dadurch gekennzeichnet, dass** der Aufnahmeschacht (18, 22) derart an das Einschubelement (23, 25; 128) angepasst ist, dass das Einschubelement nur entlang einer Richtung in den Aufnahmeschacht einführbar ist.

5. Bandscheibenprothese nach einem der vorhergehenden Ansprüche, **dadurch gekennzeichnet, dass** die Begrenzungseinrichtung (24, 26; 150, 152) derart eingerichtet ist, dass der Maximaldrehwinkel und/oder die bereitgestellte Bremskraft für die Drehbewegung mit einem Schwenkwinkel der Prothesenplatten (10) über eine, vorzugsweise stetig verlaufende, Kennlinie in Beziehung stehen.

6. Bausatz nach Anspruch 1, **dadurch gekennzeichnet, dass** sind die Bremsflächen derart ausgebildet, dass sich mit zunehmendem Drehwinkel der Abstand zwischen den Prothesenplatten (10) vergrößert.

7. Bausatz nach einem der vorhergehenden Ansprüche, **dadurch gekennzeichnet, dass** die Bremsflächen derart ausgerichtet sind, dass die Bremsfläche (24, 26; 150, 152) auf einer Prothesenplatte (10) auf der korrespondierenden Bremsfläche auf der anderen Prothesenplatte erst nach Überschreiten eines vorgegebenen Drehwinkels aufgleitet.

8. Bausatz nach einem der vorhergehenden Ansprüche, **dadurch gekennzeichnet, dass** die Bremsflächen (24, 26; 150, 152) nicht Teil des Drehlagers (34; 134) sind.

## Claims

1. A kit for constructing a spinal disc prosthesis provided for arrangement in a spinal disc compartment limited by vertebral bodies, having:
a) two prosthesis plates (10) which are arranged such that they are movable relative to one another, the exterior surfaces (12) of which are provided for contacting surfaces of opposing vertebral bodies and the interior surfaces (14) of which form corresponding bearing surfaces of a pivot bearing (34; 134) after assembly, and
b) a limiting device (24, 26; 150, 152), associated with the pivot bearing (34; 134), for limiting a rotational movement about a rotational axis (36) aligned substantially normally to the exterior surfaces (12) of the prosthesis plates (10), wherein the limiting device may be connected to the prosthesis plates (10),
**characterised in that**
the limiting device comprises braking surfaces (24, 26; 150, 152) which are aligned in such a way that, with a rotation of a prosthesis plate (10) about the rotational axis (36), a braking surface (24, 26; 150, 152) which is arranged on one of the two prosthesis plates (10) slides on a corresponding braking surface which is arranged on the other prosthesis plate, wherein the frictional resistance between the braking surfaces increases with increasing rotational angle as a result of the pressure exerted by the vertebral bodies.

2. A kit according to Claim 1, **characterised in that** the limiting device (24, 26; 150, 152) may be detachably connected to the prosthesis plates (10).

3. A kit according to Claim 1 or 2, **characterised in that** the limiting device (24, 26; 150, 152) or a part thereof is connected to an insertion element (23, 25; 128) which may be introduced into a receiving recess (18, 22) formed in one of the prosthesis plates (10).

4. A kit according to Claim 3, **characterised in that** the receiving recess (18, 22) is adapted to the insertion element (23, 25; 128) in such a way that the insertion element may only be introduced into the receiving recess in one direction.

5. A spinal disc prosthesis according to one of the preceding claims, **characterised in that** the limiting device (24, 26; 150, 152) is arranged in such a way that the maximum rotational angle and/or the braking force provided for the rotational movement are related to a pivot angle of the prosthesis plates (10) by way of a characteristic curve which extends preferably continuously.

6. A kit according to Claim 1, **characterised in that** the braking surfaces are configured in such a way that the spacing between the prosthesis plates (10) increases with increasing rotational angle.

7. A kit according to one of the preceding claims, **characterised in that** the braking surfaces are aligned in such a way that the braking surface (24, 26; 150, 152) on a prosthesis plate (10) slides on the corresponding braking surface on the other prosthesis plate only after a predetermined rotational angle has been exceeded.

8. A kit according to one of the preceding claims, **characterised in that** the braking surfaces (24, 26; 150, 152) are not part of the pivot bearing (34; 134).

## Revendications

1. Jeu de pièces pour la construction d'une prothèse de disque intervertébral qui est prévue pour être disposée dans un espace intervertébral délimité par des corps vertébraux et qui comporte :
a) deux plaques de prothèse (10) disposées de manière mobile l'une par rapport à l'autre, dont les surfaces externes (12) sont prévues pour venir s'appuyer sur des surfaces de corps vertébraux opposés et dont les surfaces internes (14) forment, après assemblage, des surfaces de palier correspondantes d'un palier de pivotement (34 ; 134), et
b) un dispositif de limitation (24, 26 ; 150, 152) associé au palier de pivotement (34 ; 134) pour limiter un mouvement de rotation autour d'un axe de rotation (36) sensiblement perpendiculaire aux surfaces externes (12) des plaques de prothèse (10), ledit dispositif de limitation pouvant être relié aux plaques de prothèse (10),
**caractérisé en ce que**
le dispositif de limitation comprend des surfaces de freinage (24, 26 ; 150, 152) qui sont orientées de façon que lors d'une rotation d'une plaque de prothèse (10) autour de l'axe de rotation (36), une surface de freinage (24, 26 ; 150, 152) qui est assujettie à une des deux plaques de prothèse (10) glisse sur une surface de freinage correspondante qui est assujettie à l'autre plaque de prothèse, la résistance de frottement entre les surfaces de freinage par suite de la pression exercée par les corps vertébraux augmentant lorsque l'angle de rotation augmente.

2. Jeu de pièces selon la revendication 1, **caractérisé en ce que** le dispositif de limitation (24, 26; 150, 152) peut être relié de manière détachable aux plaques de prothèse (10).

3. Jeu de pièces selon la revendication 1 ou 2, **caractérisé en ce que** le dispositif de limitation (24, 26 ; 150, 152) ou une partie de celui-ci est relié(e) à un élément d'insertion (23, 25 ; 128) qui peut être introduit dans un logement (18, 22) qui est ménagé dans une des plaques de prothèse (10).

4. Jeu de pièces selon la revendication 3, **caractérisé en ce que** le logement (18, 22) est adapté à l'élément d'insertion (23, 25 ; 128) de façon que l'élément d'insertion ne puisse être introduit dans le logement que suivant une direction.

5. Prothèse de disque intervertébral selon l'une des revendications précédentes, **caractérisée en ce que** le dispositif de limitation (24, 26 ; 150, 152) est conçu de façon que l'angle de rotation maximum et/ou la force de freinage fournie pour le mouvement de rotation soit en relation avec un angle de pivotement des plaques de prothèse (10) par le biais d'une courbe caractéristique, de préférence continue.

6. Jeu de pièces selon la revendication 1, **caractérisé en ce que** les surfaces de freinage sont conçues de façon que la distance entre les plaques de prothèse (10) augmente lorsque l'angle de rotation augmente.

7. Jeu de pièces selon l'une des revendications précédentes, **caractérisé en ce que** les surfaces de freinage sont orientées de façon que la surface de freinage (24, 26 ; 150, 152) sur une plaque de prothèse (10) glisse sur la surface de freinage correspondante de l'autre plaque de prothèse seulement après dépassement d'un angle de rotation prédéfini.

8. Jeu de pièces selon l'une des revendications précédentes, **caractérisé en ce que** les surfaces de freinage (24, 26 ; 150, 152) ne font pas partie du palier de pivotement (34 ; 134).
